# EUROPEAN PATENT SPECIFICATION

(11) **EP 0 666 069 B1**
(45) Date of publication and mention of the grant of the patent: **16.08.2000**
(21) Application number: 95100839.0
(22) Date of filing: 23.01.1995
(51) Int. Cl.: A61F 2/36

(54) **A prosthesis for implantation in the femur**
Eine Prothese zum Einsetzen in den Femur
Une prothèse à implanter dans le fémur

(30) Priority: 04.02.1994 IT TO940061
(43) Date of publication of application: 09.08.1995
(73) Proprietor: ITALPRO S.p.A., 20090 Opera (Milano) (IT)
(72) Inventor: Masse, Giacomo, I-12038 Savigliano (Cuneo) (IT); Bergadano, Dario, I-10127 Torino (IT)
(74) Representative: Di Francesco, Gianni

(56) References cited:
- EP-A- 0 025 814
- WO-A-81/01510
- CH-A- 507 704
- DE-A- 2 933 230
- FR-A- 2 610 822
- FR-A- 2 678 510
- US-A- 4 546 501
- US-A- 5 108 451
- US-A- 5 156 627

## Description

### Background of the invention.

The present invention relates to a femoral prosthesis of the type having a metal stem with a resting collar and a neck providing connection to the acetabulum seat.

The invention has the purpose of eliminating the causes of deterioration occurring with prosthesis implantation in the hip near the femur, particularly with non cemented prosthesis.

According to the actual knowledge, such deterioration seems to be due to bone-prosthesis mechanical action and biological transformation of the bone adjacent to the prosthesis.

It is known that metals that are typically used for this application have a modulus of elasticity which is always grater than that of the bone. It is therefore impossible to attain equal amounts of deformation under load at the metal-bone interface. As a result, relative micro-movement takes place possibly having negative consequences, such as, by way of example:
- metallosis,
- bad fastening of the stem, resulting in the prosthesis sinking in the femur,
- stem failure owing to axial offset of the prosthesis.

Practically all of the prosthesis of the above kind see for example US 5,108,451 and FR 2 610 822 that are known at present, i.e. implanted without cement addition, provide the resection of the neck of the femur, even though the latter may be healthy and mechanically valid.

### Summary of the invention.

It is an object of the present invention to provide a femoral prosthesis for implantation without cement, thereby allowing to keep the neck of the femur integral if it is not affected with disease, in order to render the transmission of forces to the metaphyseal zone of the femur more physiological, as well as maintaining the "calcar femoralis" intact for its important rib function.

Particularly, the presence of the stem of the prosthesis in the neck of the femur renders the prosthesis more resistant to torsional stresses relative to the femur axis which occur, for example, when the patient stands up from a sitting position.

In accordance with one aspect of the invention, this object is accomplished by the provision of a femoral prosthesis as claimed in claim 1.

### Brief description of the drawings.

In order that the present invention may be well understood there will now be described an embodiment thereof, given by way of example, reference being made to the accompanying drawings, in which:
- FIG. 1: is a side view of a femoral prosthesis of this invention, shown without the spherical head;
- FIG. 2: is a partially sectioned view of the prosthesis of FIG. 1 inserted in a femur in the advantageous position.

### Detailed description of the invention.

Referring to the drawings, numeral 1 designates a femoral prosthesis, of the type that is implanted without addition of cement. The femoral prosthesis is composed of a curved stem 2 generally elliptical in cross-section and downwardly tapered. The lower, distal narrower end portion 3 is rounded, while the upper end portion 4 abuts the lower surface 6 of an elliptical collar 7 of greater cross-section with respect to end portion 4.

Collar 7 is joined to a frustoconical neck 10 through a connecting portion 9. Neck 10 is of the kind known as Morse, for accommodating a spherical head 12 by means of a seat 11. In operation, spherical head 12 is coupled to a corresponding acetabulum seat forming an essential part of the prosthesis and inserted in the patient's hip (not shown).

The prosthesis is inserted in a seat 14 of the femur 13. Seat 14 is expressly obtained within the bone by milling or other method.

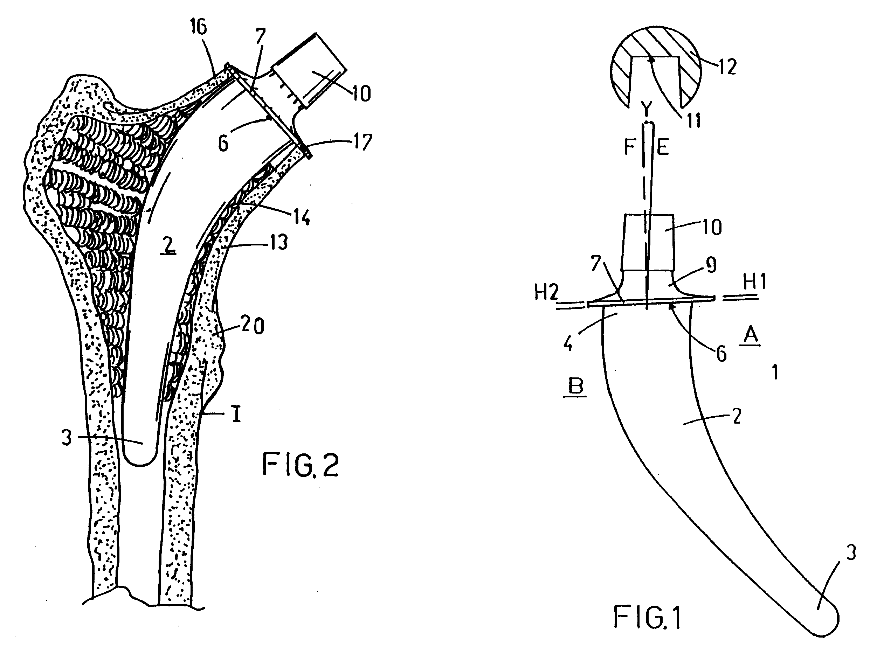

When the femur 13 is not affected with disease, the resection 17 is advantageously provided just beneath the capitellum, preserving the neck 16 integral. This is made possible by the particular shape of the prosthesis, the neck 10 of which, comprising connecting portion 9, has a very limited length. As a result, the centre of rotation of the new articulation, comprising the head 12 and the acetabulum seat, is positioned on the neck axis (E) at a distance ranging between a minimum of 19 mm and a maximum of 31 mm from the plane of resection 17 of the bone, i.e. the lower surface 6 of collar 7 resting thereon (FIG. 2).

The predetermined range of distances is a consequence of the fact that the prosthesis must necessarily adapt to various bone conformations, and therefore its size has to vary according to the height of the patient.

Line F, which is perpendicular to said lower surface 6 of collar 7, is not coaxial to axis E of neck 10, but forms an angle therewith ranging between 3 and 9 degrees.

As apparent, the lower surface 6 and the plane which is perpendicular to the plane of prosthesis 1 passing on axis E of neck 10, form a slightly acute angle facing side A where the stem 2 of prosthesis 1 extends, i.e. the medial side.

Owing to the fact that collar 7 is disposed at an angle with respect to neck 10, a better load distribution is provided on the cortical portion of the neck of the femur. Stress is concentrated on the cortical side.

Still in order to deliver the load to the cortical portion of the femur as uniformly and progressively as possible, the thickness of collar 7 is such that, besides being the minimum for supporting the loads, is not uniform throughout its perimeter. More particularly, in a preferred embodiment of the invention, a thickness H1 is provided for the collar portion facing said medial side A of the plane of the prosthesis, and a thickness H2 > H1 is provided for the collar portion facing side B diametrically opposite to side A.

As shown in FIG. 2, the stem 2 of the prosthesis of the invention is particularly not-interfering as it avoids diaphyseal cortical contacts, nor uses its distal end portion as a guiding means towards the centre of the medullar channel. In addition, reduced overall dimensions further allow conventional osteotomy in case of removal for some reason. Also, the prosthesis of this invention can be replaced by a normal temporary prosthesis so as to give the patient a greater degree of movement.

Accordingly, the length of the stem will be suitably limited in order that end portion 3 is positioned no more than 2 cm below in a distal direction relative to lower end portion I of the small trochanter of femur 13 indicated at 20. Also in this case the size of the stem is variable as a function of the size of the bone in which it is to be implanted.

The particular curved, tapered arrangement of the prosthesis according to the invention further allows to take advantage of the elasticity of the bone trabecula, not for absorbing the load during the first phase of the patient's step, but to make up for different degrees of deformability of the prosthesis itself and the bone in which it is inserted.

Therefore, attachment is provided not at metaphyseal level but in the femur neck 16, as set forth.

## Claims

1. A femoral prosthesis for the joint between the hip and the femur, comprising a curved metal stem (2) elliptical in cross-section and tapered downward, for inserting in a cavity (14) of the bone after resecting capitellum of the same, an elliptical collar (7) of greater section in comparison with the stem integral thereto, for stopping and resting said prosthesis on the plane of resection of the bone (17), a neck (10) integral with the collar and connectable to a spherical head (12); characterized in that:
a) the collar (7) has a rim protruding all around the stem (2), and
b) the centre of rotation of the prosthesis, comprising its acetabulum seat, is positioned on the axis (E) of the neck (10) at a distance ranging within a minimum of 19 mm and a maximum of 31 mm from the lover surface (6) of said collar (7).

2. A prosthesis as claimed in claim 1, characterized in that the axis (e) of the neck (10) forms an angle with the line (F) perpendicular to the lower surface (6) of the collar (7).

## Patentansprüche

1. Femorale Prothese für die Verbindung zwischen der Hüfte und dem Femur, die einen gekrümmten Metallschaft (2) umfaßt, der einen elliptischen Querschnitt aufweist und nach unten konisch zuläuft, zum Einsetzen in eine Knochenhöhlung (14) nach der Resektion des Oberschenkelkopfes, einen elliptischen Ring (7) mit größerem Querschnitt im Vergleich zum Schaft, der einstückig damit gebildet ist, damit die Prothese auf der Resektionsebene des Knochens (17) anstößt und aufsitzt, einen Hals (10) in einem Stück mit dem Ring, der mit einem kugelförmigen Kopf (12) verbunden werden kann; dadurch gekennzeichnet, daß:
a) der Ring (7) einen Rand aufweist, der um den gesamten Schaft (2) herum vorsteht, und
b) der Drehungsmittelpunkt der Prothese, der ihren Acetabulumsitz umfaßt, auf der Achse (E) des Halses (10) in einem Abstand von mindestens 19 mm bis höchstens 31 mm zur unteren Ebene (6) des Ringes (7) angeordnet ist.

2. Prothese nach Anspruch 1, dadurch gekennzeichnet, daß die Achse (E) des Halses (10) einen Winkel mit der Linie (F) senkrecht zur unteren Fläche (6) des Ringes (7) bildet.

## Revendications

1. Prothèse fémorale pour l'articulation entre la hanche et le fémur, comportant une tige métallique incurvée (2), de section transversale elliptique et s'effilant vers le bas, destinée à être insérée dans une cavité (14) de l'os après résection du condyle de celui-ci, une collerette elliptique (7) qui est d'une pièce avec la tige et de section plus large que ladite tige, destinée à assurer l'arrêt et l'appui de ladite prothèse dans le plan de résection (17) de l'os, un col (10) qui est d'une pièce avec la collerette et susceptible d'être relié à une tête sphérique (12), caractérisée en ce que :
a) la collerette (7) présente un rebord qui est en saillie tout autour de la tige (2), et
b) le centre de rotation de la prothèse, incluant sa cavité cotyloïdienne, est situé sur l'axe (E) du col (10) à une distance de la face inférieure (6) de la collerette (7) qui se situe entre une valeur minimale de 19 mm et une valeur maximale de 31 mm.

2. Prothèse fémorale selon la revendication 1, caractérisée en ce que l'axe (E) du col (10) fait un angle avec la ligne (F) qui est perpendiculaire à la surface inférieure (6) de la collerette (7).
